# EUROPEAN PATENT SPECIFICATION

(11) **EP 1 160 561 B1**
(45) Date of publication and mention of the grant of the patent: **26.10.2005**
(21) Application number: 01304781.6
(22) Date of filing: 31.05.2001
(51) Int. Cl.: G01N 1/28

(54) **Methods and apparatuses for pretreatment of metal samples**
Verfahren und Anordnungen zur Vorbehandlung von Metallproben
Procédés et appareils pour prétraiter des échantillons métalliques

(30) Priority: 01.06.2000 JP 2000163904
(43) Date of publication of application: 05.12.2001
(73) Proprietor: JFE Steel Corporation, Tokyo (JP); Abiko, Kenji, Sendai-shi, Miyagi 981-3203 (JP); Ulvac-PHI Incorporated, Chigasaki-shi, Kanagawa 253-0084 (JP); Japan Analyst Corporation, Tokyo, 141-0031 (JP)
(72) Inventor: Yasuhara, Hisao, c/o Tech. Res. Lab., Kawasaki, Chiba-shi, Chiba 260-0835 (JP); Shimura, Makoto, c/o Tech. Res. Lab., Kawasaki, Chiba-shi, Chiba 260-0835 (JP); Abiko, Kenji, Sendai-shi, Miyagi 981-3203 (JP); Iwai, Hideo, Chigasaki-shi, Kanagawa 253-0084 (JP); Niida, Takashi, c/o Japan Analyst Corporation, Tokyo 141-0031 (JP)
(74) Representative: Stebbing, Timothy Charles

(56) References cited:
- GB-A- 2 101 638
- US-A- 4 031 424
- US-A- 4 434 042
- US-A- 4 845 041
- PATENT ABSTRACTS OF JAPAN vol. 2000, no. 02, 29 February 2000 (2000-02-29) & JP 11 316220 A (KAWASAKI STEEL CORP;ABIKO KENJI; NIPPON ANALYST KK), 16 November 1999 (1999-11-16)
- SHEVCHENKO N B ET AL: "GRANULAR THIN FILM DEPOSITION BY SIMULTANEOUS SPARK EROSION AND SPUTTERING" JOURNAL OF APPLIED PHYSICS, AMERICAN INSTITUTE OF PHYSICS. NEW YORK, US, vol. 81, no. 8, PART 2B, 15 April 1997 (1997-04-15), pages 5564-5566, XP000701283 ISSN: 0021-8979

## Description

This invention relates to methods and apparatuses for pretreatment of metal samples performed prior to the analysis of elements in metals.

Trace elements in metals, such as carbon, oxygen, nitrogen and sulfur in steel, have a considerable effect on the ductility and workability of the metals. Consequently, the accurate determination of such elements has been important. For example, in the quantitative determination of oxygen in steel by fusion analysis, a sample is introduced into a reaction chamber (graphite crucible) and is heated under a carrier gas flow to extract trace elements (CO and CO₂) into the carrier gas. The extracted trace element (oxygen) is then quantitatively determined by, for instance, an infrared-ray absorbing device.

As metal materials have recently become much purer than before, the demand is increasing for higher analytical precision for the analysis of trace elements. For instance, an analysis error has to be ± 0.5 ppm or less for the quantitative determination of oxygen in steel. In order to satisfy this requirement, it is important to remove substances adsorbed on the surface of metals, in other words, contaminants on the surface, oxides generated by oxidation with the air, and other substances, before quantitative analysis is performed. Japanese Unexamined Patent Application Publication Nos. 8-211043, 10-73586 and 11-316220 disclose pretreatment methods and apparatuses for metal samples to perform element analysis of metals.

The pretreatment methods generally introduce inert gas (for instance, argon gas) into a pretreatment chamber where a cathode as a metal sample is surrounded with an anode, thus removing contaminants on the surface of the metal sample by sputtering with cations.

The methods mentioned above remove contaminants or oxides stuck and/or adsorbed on the surface of metal samples by sputtering, thus decreasing analysis errors due to the contaminants or oxides. However, the present inventors discovered that, for instance, the oxygen level increases and becomes unstable when a sequence of analyses, including the pretreatment by sputtering mentioned above, is repeated. The present inventors examined the causes for re-contamination and discovered that an electrode surrounding a sample is heated in a sputtering step, so that a substance adsorbed on a counter electrode, such as water, is removed and the cleaned surface of the sample is re-oxidized by sputtering. Other causes of re-contamination include re-adsorption of sputtering material on the surface of samples, and re-oxidation of the surface due to moisture or the like as an impurity in atmospheric gas.

US-A-4 845 041 discloses a method of analysis of metal sample by sputtering which includes a step of cooling a cathode plate which firmly contacts the sample. A pre-treatment step to clean the sample surface is also disclosed. This document is in accordance with the preamble of each independent claim.

US-A-4 031 424 discloses a method of sputtering material from a cathode to provide a coating on a substrate. Both the anode and the cathode (target) are cooled:

According to a first aspect of the present invention there is provided a pretreatment method for element analysis of a metal sample, comprising removing contaminants on a surface of the metal sample by sputtering, characterised in that at least one counter electrode for sputtering arranged to counter an electrode holding the metal sample is cooled.

According to a second aspect of the present invention there is provided a pre-treatment apparatus for element analysis of a metal sample, comprising:
an electrode for holding a metal sample;
at least one counter electrode arranged to counter the electrode holding the metal sample for sputtering; and
a pre-treatment chamber for storing the electrode holding the metal sample, each counter electrode and the metal sample under an inert gas atmosphere; characterised by:
   a cooling device for cooling at least one counter electrode.

Embodiments of the invention may provide pretreatment methods and apparatuses for metal samples that prevent the re-contamination of sample surfaces, such as by re-oxidation, due to sputtering. The invention provides accurate analytical levels even when sputtering is repeated.

The invention relates to pretreatment methods for element analysis of metal samples. Contaminants on the surface of metal samples are removed by sputtering, while at least one counter electrodes is being cooled.

In embodiments, it is preferable in the pretreatment method that the metal sample is held at the side of a cathode and a plurality of anodes face the cathode, and sputtering is carried out while at least one of the anodes is being cooled. In embodiments of the methods, the polarity of electrodes may be reversed. In other embodiments of the methods, the metal sample may be held at the side of an anode and a plurality of cathodes may face the anode, and sputtering may be carried out while at least one of the cathodes is cooled.

Furthermore, it is preferable that an element to be analyzed in the metal sample is at least one of carbon, oxygen, nitrogen and sulfur in any embodiment of the methods mentioned above.

It is also preferable that element analysis for metal samples is at least one of fusion analysis and combustion analysis in any embodiment of the methods mentioned above.

Accordingly, the pretreatment methods of the invention are preferably applied when the element analysis for a metal sample is at least one of fusion analysis and combustion analysis, and when an element to be analyzed is at least one of carbon, oxygen, nitrogen and sulfur.

The invention also provides pretreatment apparatuses for the element analysis of metal samples. Embodiments have a cathode for holding the metal sample, anodes arranged to face the cathode to carry out sputtering, and a pretreatment chamber for storing the electrodes and the metal sample under an inert gas atmosphere. The apparatuses are provided with a cooling device to cool at least one of the anodes.

Furthermore, in the above-described apparatuses, the polarity of electrodes may be reversed. In such embodiments, the apparatuses can include an anode for holding a metal sample, cathodes arranged to face the anode to carry out sputtering, and a pretreatment chamber for storing the electrodes and the metal sample under an inert gaseous atmosphere. The apparatuses may be provided with a cooling device to cool at least one of the cathodes.

In the drawings:
FIG. 1 is a schematic view of an exemplary embodiment of the apparatuses of the invention for analyzing trace elements in metals;
FIG. 2 is a schematic view of another exemplary embodiment of the apparatuses of the invention for analyzing trace elements in metals;
FIG. 3 is a cross-sectional view showing the structure of a pretreatment apparatus for metal samples according to the invention; and
FIG. 4 is a cross-sectional view showing another exemplary structure of a pretreatment apparatus for metal samples according to the invention.

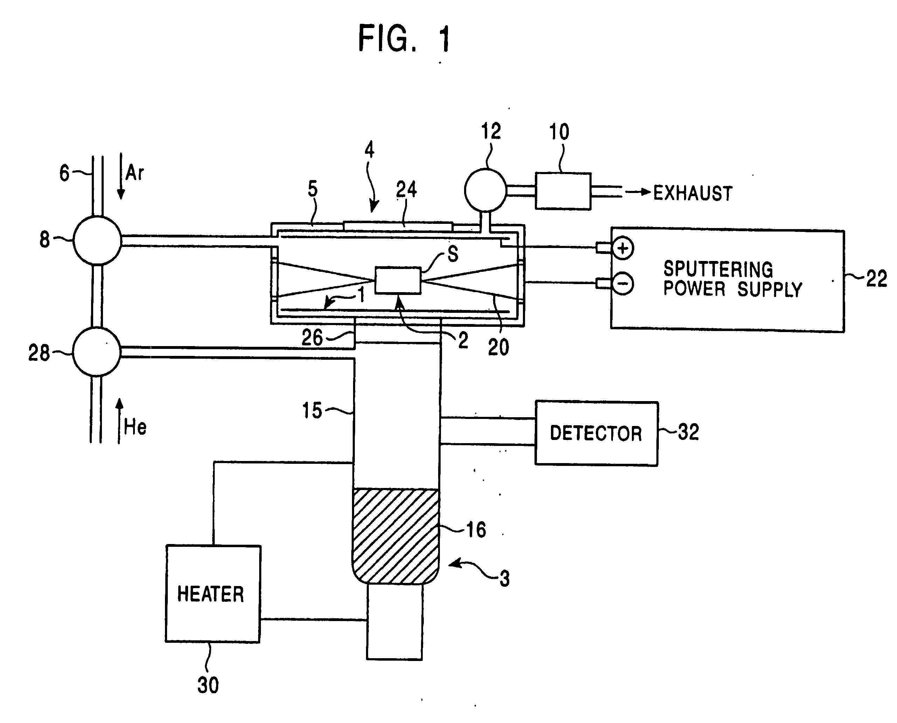

FIG. 1 schematically shows an analyzer, according to the invention. The analyzer includes a pretreatment apparatus, for trace elements in metals for use in embodiments of the invention. In FIG. 1, a metal sample S is held at the side of a cathode. As shown in the figure, or in Japanese Unexamined Patent Application Publication No. 11-316220, an analyzer 3 has a pretreatment apparatus 4. The pretreatment apparatus 4 removes contaminants on the surface of the sample S. Specifically, the pretreatment apparatus 4 has a sample chamber 5; a sample holder 20 for supporting the sample S in the sample chamber 5; a metal anode 1 arranged at an inner wall of the sample chamber 5; a gas source, such as an argon gas source 6, a valve 8, an exhaust pump 10 and a valve 12, to introduce sputtering gas, such as, e.g., argon gas, into the sample chamber 5 and to generate a sputtering atmosphere; and a power supply 22 for sputtering.

The pretreatment apparatus 4 can remove contaminants from the surface of the sample by colliding argon, which is ionized by negatively charging a cathode 2, including the sample S and the sample holder 20, and by positively charging the anode 1. The sample S is introduced into the sample chamber 5 through a sample input port 24.

The sample S, from which contaminants are removed by the pretreatment apparatus 4, is introduced into a reaction chamber 15 of the analyzer 3 through a shutter 26 for element analysis of the sample S. For instance, for oxygen analysis, the sample S is heated in helium gas in a graphite crucible 16 by a heater 30, and generated CO gas and/or CO₂ gas is quantitatively determined by a detector 32, such as an infrared-ray absorbing device or any other suitable device.

Additionally, in the invention, the surface of metal samples is not re-contaminated when the above-described analyzer quantitatively determines trace elements in metals. Therefore, when contaminants are removed from the surface of metal samples by sputtering, an electrode surrounding the metal sample S is cooled.

A targeted cooling site is a counter electrode that is heated, or where the temperature increases, during sputtering. In the above-described apparatus, the metal anode 1 at the inner wall of the sample chamber 5 is targeted. However, it is unnecessary to cool the entire anode 1, and cooling may be limited only to substantially heated portions. The cooling temperature may be set at a level to keep down the temperature of an electrode so as not to remove substances adsorbed to an anode, such as water. Generally, the electrode may be set at a temperature of about 50°C or below. Due to such cooling, the re-scattering of contaminants removed by sputtering can be controlled, thus preventing the contaminants from readhering to the surface of a sample. Moreover, in order to prevent the re-adsorption of sputtering materials, or the adsorption of impurities in atmospheric gas, the anode 1 is cooled by liquid nitrogen or the like to function as a shroud.

Moreover, when a metal sample is held at the side of a cathode as shown in FIG. 1, cation sputtering is performed. In the invention, glow discharge, plasma etching or the like may be used for cation sputtering. Glow discharge is most preferable for use in the invention.

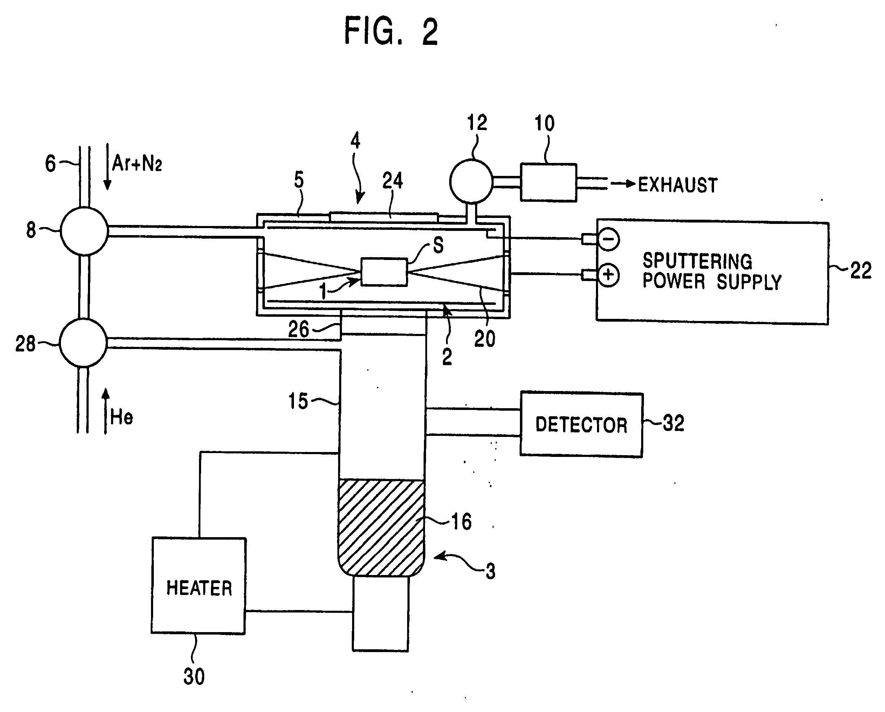

FIG. 2 schematically shows an analyzer 3, including a pretreatment apparatus 4, for trace elements in metals for use in the invention. FIG. 2 is different from FIG. 1, in that in FIG. 2 a metal sample is held at the side of an anode. In this embodiment, sputtering is performed with anions. Arc discharge, glow discharge or the like may be used for anion sputtering in the invention. Arc discharge is preferably used in the invention.

When contaminants are removed by sputtering with arc discharge, the metal sample becomes an anode and cathodes are arranged around the sample. The gas source 6 is, for instance, a mixed gas of argon gas and nitrogen or gaseous nitrogen. Other features of the apparatus of FIG. 2 are the same as those of FIG. 1. This apparatus prohibits not only oxygen re-contamination due to oxygen analysis, but also hydrocarbon re-contamination due to a hydrocarbon material from carbon analysis or hydrogen analysis.

The invention can use the above-described apparatuses shown in FIGS. 1 and 2, and, for example, combustion analysis, GDS, emission spectrometry, X-ray diffraction methods, in addition to oxygen analysis by fusion analysis described above, for element analysis. These methods may be selected depending on the element(s) to be analyzed. For instance, for carbon and sulfur analysis, combustion analysis or fusion analysis may be selected.

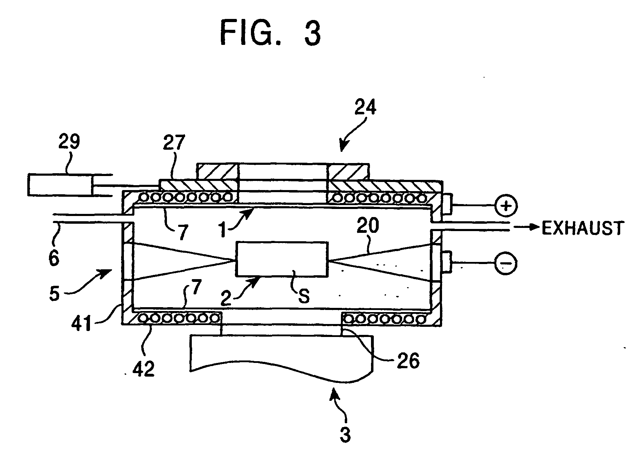

In an embodiment of the pretreatment apparatus described above, a cooling device is provided to compulsively cool (actively cool) the counter electrodes of an electrode for holding a metal sample, as described below, so as to cool the electrodes. FIG. 3 is a cross-sectional view of an exemplary pretreatment apparatus including the cooling device. The anodes 1 include metal plates 7 as the counter electrodes of a metal sample. The metal plates 7 are cooled by running a cooling pipe in a wall body 41 of the sample chamber 5 that supports the metal plates 7. The metal plates 7 are arranged at top and bottom wall surfaces, sandwiching a cathode therebetween. However, in some embodiments, it is preferable to provide metal plates 7 at four wall surfaces, including left and right wall surfaces. Further, in some embodiments, it is preferable to provide an anode at six wall surfaces of the sample chamber 5, thus more evenly cleaning the surface of metal samples. Other structural characteristics of the pretreatment apparatus are basically the same as those of embodiments described above. In this example, the sample input port 24 has a slide gate 27 and a cylinder 29 to shift the slide gate 27 right and left to open and close the sample input port 24.

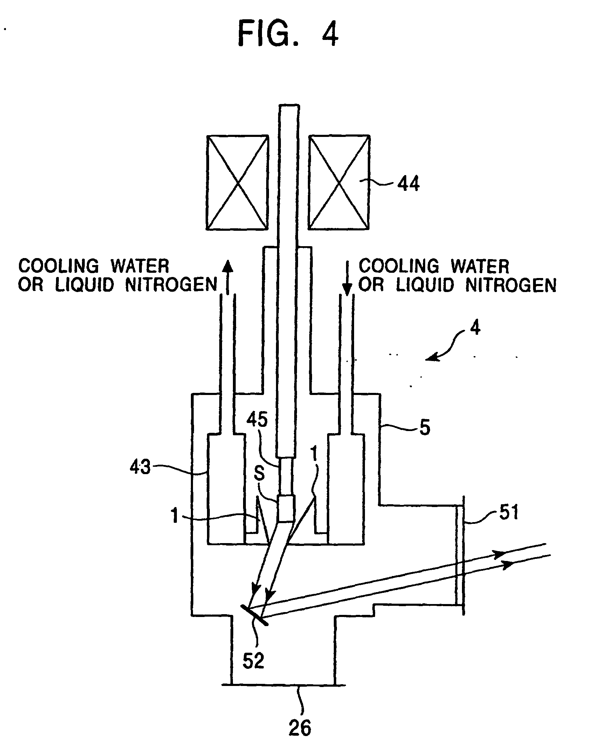

FIG. 4 is a cross-sectional view showing another exemplary structure of the pretreatment apparatus 4 according to the invention. In this example, a sample S is hung in the sample chamber 5 by a magnetic body 45 that is magnetized by an exciter 44. Anode portions 1 are arranged around the sample S, and a selected level of high voltage is applied to the anode portions 1 and the sample S.

Moreover, cooling members 43, such as cooling boxes, are arranged in the sample chamber 5 so as to surround the back of the anode portions 1. A cooling medium, such as cooling water or liquid nitrogen, is circulated in the cooling members 43. Thus, the cooling members 43 indirectly cool the anode portions 1. Additionally, the anode portions 1 comprise a thick object, so that the anode portions 1 may be directly cooled by providing a hollow space inside them and circulating cooling water or liquid nitrogen in the anode portions 1.

As described above, it is preferable that the cooling device includes cooling boxes arranged at the counter electrodes of a metal sample, or joined to the back thereof.

In the example, the pretreated sample S is also introduced into an analyzer body (not shown in FIG. 3) through a shutter 26 provided at the bottom of the pretreatment apparatus 4. Therefore, the sample is pretreated within one system without being re-contaminated, and is analyzed. Additionally, the sample S can be directly monitored during a pretreatment process by a mirror 52 provided at the lower diagonal direction of the sample S and also a monitoring window 51 arranged at the side of the sample chamber 5 in the example.

Table 1 below shows data indicating the correlation between the number of repetitions of analysis and oxygen levels (mass ppm) when steel samples having different oxygen contents were continuously analyzed by fusion-infrared ray analysis with the pretreatment apparatus shown in FIG. 4. As shown in Table 1, a sample was not re-contaminated even when element analysis was continuously repeated three times or more by a pretreatment apparatus in which an anode was cooled by liquid nitrogen in accordance with the invention. Accordingly, oxygen levels did not rise sharply. In contrast, when element analysis was repeated three times or more by a conventional pretreatment apparatus in which an anode was not cooled, oxygen levels increased by 1 to 1.6 ppm in mass, and it was difficult to obtain an accurate oxygen level. The above-described results were similarly found when the oxygen content in steel is high (sample A) and also low (sample B).

**Table 1**

| | Sample A | | Sample B | |
|---|---|---|---|---|
| Number of repetitions | Conventional Example | Invention | Conventional Example | Invention |
| 1 | 16.0 | 15.6 | 3.4 | 3.3 |
| 2 | 16.2 | 15.5 | 3.5 | 3.2 |
| 3 | 16.4 | 16.1 | 4.1 | 3.0 |
| 4 | 17.6 | 15.9 | 4.9 | 3.1 |
| 5 | 17.6 | 15.7 | 5.0 | 3.0 |
| (mass ppm) | | | | |

Oxygen analysis is described in detail above with reference to exemplary embodiments of the invention. However, the invention is not limited to the above embodiments, and is widely applicable for preventing re-contamination by cooling a counter electrode. Although the re-contamination of metal samples is prevented, contaminants sometimes accumulate on cooling electrodes. Such accumulated contaminants can be removed by baking a contaminated electrode regularly or irregularly. The invention can prevent the re-adsorption of not only water as a contaminant, but also other materials such as SO₂, CO₂, N₂ and hydrocarbons as materials accumulated on an anode throughout a pretreatment step.

Moreover, the analyzer is not limited to the ones shown in the exemplary embodiments, and any suitable analyzer can be used as long as it can directly receive a pretreated sample and quantitatively determine trace elements. In the embodiments mentioned above, glow discharge sputtering is used. However, arc discharge sputtering is also applicable, and in this case, a sample is an anode as shown in FIG. 2 and cools cathodes as counter electrodes.

The invention can prevent the recontamination of metal samples during a pretreatment process by cooling at least one counter electrode when contaminants on the surface of metal samples are removed by sputtering prior to the element analysis of metals. Therefore, even if pretreatment is continuously carried out, the analytical levels of trace elements may be obtained with good reproducibility.

## Claims

1. A pretreatment method for element analysis of a metal sample, comprising removing contaminants on a surface of the metal sample by sputtering, **characterised in that** at least one counter electrode for sputtering arranged to counter an electrode holding the metal sample is cooled.

2. The pretreatment method according to claim 1, wherein the metal sample is at a side of a cathode and a plurality of anodes face the cathode, and at least one of the anodes is cooled for sputtering.

3. The pretreatment method according to claim 1, wherein the metal sample is at a side of an anode and a plurality of cathodes face the anode, and at least one of the cathodes is cooled for sputtering.

4. The pre-treatment method according to claim 1, 2, or 3, comprising analyzing an element in the metal sample selected from the group consisting of carbon, oxygen, nitrogen and sulphur.

5. The pretreatment method according to claim 1, 2, 3 or 4, wherein the element analysis of the metal sample is by fusion analysis or combustion analysis.

6. A pre-treatment apparatus for element analysis of a metal sample, comprising:
an electrode for holding a metal sample;
at least one counter electrode arranged to counter the electrode holding the metal sample for sputtering; and
a pre-treatment chamber for storing the electrode holding the metal sample, each counter electrode and the metal sample, under an inert gas atmosphere; **characterised by**:
a cooling device for cooling at least one counter electrode.

7. The pretreatment apparatus according to claim 6, comprising a plurality of the counter electrodes arranged to counter the electrode holding the metal sample, and the cooling device cools at least one of the counter electrodes.

8. The pre-treatment apparatus according to claim 6 or 7, wherein the electrode holding the metal sample is a cathode and each counter electrode is an anode.

9. The pre-treatment apparatus according to claim 6 or 7, wherein the electrode holding the metal sample is an anode and each counter electrode is a cathode.

## Patentansprüche

1. Vorbehandlungsverfahren für die Elementanalyse einer Metallprobe, aufweisend das Entfernen von kontaminierenden Partikeln auf einer Oberfläche der Metallprobe durch Sputtern, **dadurch gekennzeichnet, dass** mindestens eine Gegenelektrode für das Sputtern, welche dafür angeordnet ist, einer Elektrode, welche die Metallprobe hält, entgegenzuwirken, gekühlt wird.

2. Vorbehandlungsverfahren nach Anspruch 1, wobei die Metallprobe an einer Seite einer Kathode ist und eine Anzahl an Anoden zur Kathode gerichtet sind und mindestens eine der Anoden für das Sputtern gekühlt wird.

3. Vorbehandlungsverfahren nach Anspruch 1, wobei die Metallprobe an einer Seite einer Anode ist und eine Anzahl an Kathoden zur Anode gerichtet sind und zumindest eine der Kathoden für das Sputtern gekühlt wird.

4. Vorbehandlungsverfahren nach Anspruch 1, 2 oder 3, aufweisend das Analysieren eines Elements in der Metallprobe, ausgewählt aus der Gruppe, die aus Kohlenstoff, Sauerstoff, Stickstoff und Schwefel besteht.

5. Vorbehandlungsverfahren nach Anspruch 1, 2, 3 oder 4, wobei die Elementanalyse der Metallprobe durch Fusionsanalyse oder Verbrennungsanalyse ausgeführt wird.

6. Vorbehandlungsvorrichtung für die Elementanalyse einer Metallprobe, aufweisend:
eine Elektrode zum Halten einer Metallprobe;
mindestens eine Gegenelektrode, welche dafür angeordnet ist, der Elektrode, welche die Metallprobe für das Sputtern hält, entgegenzuwirken; und
eine Vorbehandlungskammer zum Lagern der Elektrode, welche die Metallprobe hält, jeder Gegenelektrode und der Metallprobe in einer Inertgasatmosphäre; **gekennzeichnet durch**:
eine Kühlvorrichtung zum Kühlen zumindest einer Gegenelektrode.

7. Vorbehandlungsvorrichtung nach Anspruch 6, aufweisend eine Anzahl an Gegenelektroden, die dafür angeordnet sind, der Elektrode, welche die Metallprobe hält, entgegenzuwirken, wobei die Kühlvorrichtung zumindest eine der Gegenelektroden kühlt.

8. Vorbehandlungsvorrichtung nach Anspruch 6 oder 7, wobei die Elektrode, welche die Metallprobe hält, eine Kathode ist und jede Gegenelektrode eine Anode ist.

9. Vorbehandlungsvorrichtung nach Anspruch 6 oder 7, wobei die Elektrode, welche die Metallprobe hält, eine Anode ist und jede Gegenelektrode eine Kathode ist.

## Revendications

1. Procédé de prétraitement pour l'analyse d'éléments d'un échantillon métallique, comprenant l'élimination de contaminants sur une surface de l'échantillon métallique par pulvérisation, **caractérisé en ce qu'**au moins une contre-électrode de pulvérisation agencée pour s'opposer en polarité à une électrode en contact avec l'échantillon métallique est refroidie.

2. Procédé de prétraitement selon la revendication 1, dans lequel l'échantillon métallique est sur un côté d'une cathode et une pluralité d'anodes sont face à la cathode, et au moins une des anodes est refroidie pour la pulvérisation.

3. Procédé de prétraitement selon la revendication 1, dans lequel l'échantillon métallique est sur un côté d'une anode et une pluralité de cathodes sont face à l'anode, et au moins une des cathodes est refroidie pour la pulvérisation.

4. Procédé de prétraitement selon la revendication 1, 2 ou 3, comprenant l'analyse d'un élément dans l'échantillon métallique choisi dans le groupe comprenant le carbone, l'oxygène, l'azote et le soufre.

5. Procédé de prétraitement selon la revendication 1, 2, 3 ou 4, dans lequel l'analyse d'éléments d'échantillon métallique est une analyse par fusion ou une analyse par combustion.

6. Appareil de prétraitement pour l'analyse d'éléments d'un échantillon métallique, comprenant :
une électrode pour maintenir un échantillon métallique ;
au moins une contre-électrode agencée pour s'opposer en polarité à l'électrode en contact avec l'échantillon métallique pour la pulvérisation ; et
une chambre de prétraitement pour confiner l'électrode en contact avec l'échantillon métallique, chaque contre-électrode et l'échantillon métallique sous une atmosphère de gaz inerte ; **caractérisé en ce qu'**il comprend :
un dispositif de refroidissement pour refroidir au moins une contre-électrode.

7. Appareil de prétraitement selon la revendication 6, comprenant une pluralité de contre-électrodes agencées pour s'opposer en polarité à l'électrode en contact avec l'échantillon métallique, et le dispositif de refroidissement refroidit au moins une des contre-électrodes.

8. Appareil de prétraitement selon la revendication 6 ou 7, dans lequel l'électrode en contact avec l'échantillon métallique est une cathode et chaque contre-électrode est une anode.

9. Appareil de prétraitement selon la revendication 6 ou 7, dans lequel l'électrode en contact avec l'échantillon métallique est une anode et chaque contre-électrode est une cathode.
